Europäisches Patentamt

European Patent Office

Office européen des brevets

(19)

(11) Veröffentlichungsnummer : **0 115 580 B1**

# (12) EUROPÄISCHE PATENTSCHRIFT

(45) Veröffentlichungstag der Patentschrift :
29.07.87

(51) Int. Cl.⁴ : **A 61 B 17/06**

(21) Anmeldenummer : 83111143.0

(22) Anmeldetag : 08.11.83

(54) **Packung für chirurgisches Nahtmaterial.**

(30) Priorität : 14.12.82 DE 3246162

(43) Veröffentlichungstag der Anmeldung :
15.08.84 Patentblatt 84/33

(45) Bekanntmachung des Hinweises auf die Patenterteilung : 29.07.87 Patentblatt 87/31

(84) Benannte Vertragsstaaten :
AT BE CH DE FR IT LI LU NL SE

(56) Entgegenhaltungen :
EP-A- 0 046 518
DE-C- 1 288 737

(73) Patentinhaber : **INTERMEDICAT GMBH**
**Gerliswilstrasse 74**
**CH-6020 Emmenbrücke (CH)**

(72) Erfinder : **Riesenberger, Hermann**
**Schwalbenweg 3**
**D-6440 Bebra-Weitrode (DE)**
Erfinder : **Schwäble, Horst**
**Knüllstrasse 23**
**D-3508 Melsungen (DE)**

(74) Vertreter : **von Kreisler, Alek, Dipl.-Chem. et al**
**Deichmannhaus am Hauptbahnhof**
**D-5000 Köln 1 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft eine Packung für chirurgisches Nahtmaterial, mit einem abdichtend verschlossenen Behälter aus einem wannenförmigen ersten Folienteil und einem das erste Folienteil entlang gegeneinanderliegender Randbereiche als aufreißbarer Deckel verschließenden zweiten Folienteil, mit an den Folienteilen vorgesehenen Aufreißlaschen und mit einer in dem Behälter angeordneten, die wesentliche Menge des Nahtmaterials enthaltenden Faltkarte, die eine an dem den Aufreißlaschen abgewandten Rand mit dem zweiten Folienteil fest verbundene Lasche mit einem Schlitz zum Festlegen eines Abschnitts des aus der Faltkarte herausragenden Nahtmaterials aufweist.

Eine bekannte Packung dieser Art (DE-C-3 032 037) weist einen aus zwei miteinander verschweißten Folienteilen bestehenden Behälter auf, der eine Faltkarte als Nahtmaterialträger enthält. Das Nahtmaterial in Form eines Fadens ist im Innern der Faltkarte angeordnet und ragt aus einer Öffnung der Faltkarte heraus. Das freie Fadenende ist in einem Schlitz einer Lasche festgelegt, die von der Bodenwand der Faltkarte nach außen absteht und mehrere querlaufende Faltlinien aufweist. Das Ende der Lasche ist mit dem den Deckel bildenden zweiten Folienteil verbunden. Wird der Deckel aufgerissen, so stellt sich die gefaltete Lasche hoch, während die Faltkarte in der von dem zweiten Folienteil gebildeten Wanne verbleibt. Das in einem Schlitz der Lasche festgelegte Fadenende bzw. die an dem Fadenende befestigte Nadel kann ergriffen werden, um den Faden in seiner gesamten Länge aus der Faltkarte herauszuziehen. Die bekannte Packung erfordert für die einstückig mit der Faltkarte verbundene Lasche zusätzliches Material und eine relativ komplizierte Faltung. Im gefalteten Zustand überdeckt die Lasche einen Teil der Faltkarte. Die Lasche bedarf einer zusätzlichen Festlegung auf der Faltkarte und überdeckt deren Bedruckung, die auf der betreffenden Seite durch das zweite Folienteil hindurch nicht vollständig sichtbar ist. Außerdem wird die Stärke des aus der Faltkarte und der gefalteten Lasche bestehende Paketes an der Stelle der Lasche gegenüber den übrigen Bereichen erhebliche vergrößert.

Bei einer weiteren bekannten Nahtmaterialpackung (DE-C-2 532 992) ist eine Lasche, die in der Oberseite der Faltkarte vorgesehen ist, zwischen den Aufreißlaschen der Folienpackung eingeschweißt. Beim Aufreißen der Packung wird die Oberseite der Faltkarte zusammen mit dem oberen Folienteil des Behälters aufgerissen, so daß das auf der darunter befindlichen Platte der Faltpackung liegende Nahtmaterialende freigelegt wird, jedoch ohne aus der Faltkarte herausgehoben zu werden.

Der Erfindung liegt die Aufgabe zugrunde, eine Packung der eingangs genannten Art zu schaffen, bei der die Lasche zum Festlegen und Darreichen des Fadenendes keine störende Verdickung bzw. Überlappung im Innern des Behälters bildet.

Zur Lösung dieser Aufgabe ist erfindungsgemäß vorgesehen, daß die ungefaltete Lasche aus einem ausgestanzten Teil der nach dem Aufreißen des zweiten Folienteils freiliegenden Oberseite der Faltkarte besteht und mit ihrem den Aufreißlaschen abgewandten Rand an dem zweiten Folienteil derart befestigt ist, daß sie sich beim Aufreißen des zweiten Folienteils aus der Oberseite der Faltkarte heraus hochstellt.

Bei der erfindungsgemäßen Packung besteht die Lasche, an der das zu ergreifende Fadenende festlegt ist, aus einem ausgestanzten Teil der Oberseite der Faltkarte. Beim Aufreißen des zweiten Folienteiles wird die Lasche aus der Oberseite der Faltkarte herausgehoben, wobei das Fadenende mit angehoben wird. Das Fadenende kann nun von der frei aufragenden Lasche angenommen werden. Im verpackten Zustand fügt sich die Lasche passend in eine entsprechende Ausnehmung der Oberseite der Faltkarte ein. Wird die Oberseite mit einer Bedruckung versehen, die durch das durchsichtige erste Folienteil hindurch sichtbar ist, dann kann diese Bedruckung sich auch über die Lasche erstrecken, die in diesem Zustand ja einen Teil der Oberseite der Faltkarte bildet.

Gemäß einer bevorzugten Weiterbildung der Erfindung ist die Lasche über schmale abreißbare Stege mit der Oberseite der Faltkarte verbunden. Diese Stege sind vorzugsweise an dem den Aufreißlaschen der Packung abgewandten Rand der Faltkarte angeordnet. Beim Aufreißen des zweiten Folienteils reißen auch die Stege ab, die die Lasche mit der Oberseite der Faltkarte verbinden. Die Lasche ist dann nur noch an dem geöffneten Deckel befestigt, von dessem Fußbereich sie absteht, so daß das Fadenende auf beiden Seiten der Lasche ergriffen werden kann. Hierbei kann die Lasche auch abgerissen werden. Da das Öffnen einer Packung mit chirurgischem Nahtmaterial in der Regel mit Gummihandschuhen erfolgt, die keine sehr genauen und feinfühligen Handgriffe zulassen, ist es wichtig, daß die aus einem einlagigen Kartonstück bestehende Lasche, an der das Fadenende festgelegt ist, von beiden Seiten zwischen Daumen und Zeigefinger ergriffen werden kann, um das Fadenende sicher in die Hand zu bekommen. Die abgerissene Lasche bildet eine Halterung für den Faden. Bei Mehrfadenpackungen sind sämtliche Fäden an dieser Lasche festgelegt, so daß im Operationssaal die Packung nach dem Abreißen der Lasche fortgeworfen werden kann. Von der Lasche, in deren Schlitz bzw. Schlitzen die Fäden festgelegt sind, können diese Fäden einzeln nach Bedarf abgezogen werden. Damit entfallen die bei Mehrfadenpackungen sonst üblichen zusätzlichen Halterungen, die der Packung zusammen mit den Fäden entnommen werden.

Gemäß einer vorteilhaften Weiterbildung der Erfindung ist vorgesehen, daß die Oberseite der Faltkarte mit dem den Aufreißlaschen abgewandten Rand frei übersteht und daß der überstehende Bereich die Vertiefung der aus dem ersten Folienteil gebildeten Wanne überragt und im wesentlichen nur im Bereich der Lasche mit dem zweiten Folienteil verbunden ist. Dadurch wird erreicht, daß der überstehende Rand der Faltkarte zwischen den beiden Folienteilen festgelegt ist, so daß die gesamte Faltkarte in dem Behälter fixiert ist und sich insbesondere während des Aufreißens nicht verschieben kann. Im Bereich des überstehenden Randes ist die Faltkarte mit dem zweiten Folienteil des Behälters an der Stelle der Lasche sogar durch Schweißung oder Klebung fest verbunden. Durch diese Verbindung im Fußbereich der Lasche erfolgt beim Aufreißen des Behälters das Hochstellen der Lasche.

Vorzugsweise ist die Lasche wenigstens annähernd halbkreisförmig ausgebildet und in der Mitte der Länge der Faltkarte angeordnet.

Die Faltkarte kann unterhalb ihrer Oberseite eine den Raum zur Aufnahme des Nahtmaterials nach oben begrenzende Zwischenwand aufweisen, die unter der Lasche eine Öffnung für den Durchtritt des an der Lasche festgelegten Nahtmaterials aufweist. Der eigentliche Aufnahmeraum für das Nahtmaterial wird also nicht durch die Oberseite der Faltkarte begrenzt, sondern durch die Zwischenwand. Erst nach dem Hochstellen der Lasche wird die in der Zwischenwand vorgesehene Öffnung, durch die das Nahtmaterial aus dem Aufnahmeraum herausgezogen wird, freigelegt. Die Lasche weist über dieser Öffnung der Zwischenwand eine weitere Öffnung oder einen Schlitz zum Hindurchführen und Festlegen des Fadens auf.

Wenn die Lasche hochgestellt worden ist, wird der Faden an der Austrittsstelle aus dem Aufnahmeraum in Richtung auf diejenige Schmalseite der Faltkarte gezogen, von der die Lasche abgezogen worden ist. Um ein Ausreißen der Faltkarte an dieser Stell zu vermeiden, und zur Vermeidung übermässiger Reibung am Faden erstreckt sich die Öffnung in der Zwischenwand zweckmäßigerweise bis in die benachbarte Schmalseite der Faltkarte hinein. Auf dem in der Schmalseite befindlichen Bereich der Öffnung wird dann schließlich das Nahtmaterial abgezogen.

Die Lasche weist mindestens eine ausgestanzte Zunge auf, deren Kante in einen Rand der Lasche ausläuft. Diese Zunge bildet also einen bogenförmigen Schlitz, in den der Faden eingeschoben und festgeklemmt werden kann.

Gemäß einer bevorzugten Ausführungsform der Erfindung ist die Lasche mit zwei durch eine Aussparung voneinander getrennten Beinen an dem zweiten Folienteil befestigt. An die äußeren Enden der Beine schließen sich die zerreißbaren dünnen Stege an, an denen die Lasche mit der Oberseite verbunden ist. Infolge der Aussparung zwischen den Beinen kann die Lasche leicht und definiert von dem zweiten Folienteil abgerissen werden. Die Aussparung dient ferner zum Hindurchstecken des an der Lasche befestigten Fadenabschnitts und zum Einschieben dieses Fadenabschnitts in einen Schlitz, der von dieser Aussparung abgeht. Vorzugsweise sind an der Lasche mindestens zwei Zungen vorgesehen, von denen eine in die Aussparung auslaufende Kante aufweist und somit einen entsprechenden Schlitz bildet.

Die Packung kann für Nahtmaterial verwendet werden, das aus einem einzigen Faden besteht, oder auch für Nahtmaterial, das aus mehreren separaten Fäden besteht. Sind mehrere Fäden vorgesehen, so werden diese nebeneinander und mit untereinander gleichem Verlauf an den Schlitzen der Lasche festgelegt.

Obwohl das Fadenende an der Lasche festgelegt werden kann, ist es bei Verwendung von Fäden, die an ihrem Ende eine Nadel aufweisen, auch möglich, diese Nadel in Schlitze der Oberseite der Faltkarte einzuschieben, wobei diese Schlitze außerhalb des Bereichs der Lasche liegen. Der Faden wird hierbei zweckmäßigerweise durch Schlitze, die an der Lasche vorgesehen sind, aus dem Innern der Faltkarte nach außen geführt, jedoch erfolgt die Festlegung des Fadenendes (bzw. der Nadel) nicht unmittelbar an der Lasche. Auch hierbei stellt sich die Lasche beim Aufreißen der Packung auf, so daß der Faden über den Rand der Lasche gezogen wird und auf diese Weise ebenfalls leicht mit der Hand bzw. einem Nadelhalter ergriffen werden kann, um die an ihm befestigte Nadel von der Faltkate abzuziehen.

Zweckmäßigerweise ist in Längsrichtung der Faltkarte vor und hinter der Lasche jeweils ein Schlitz zum Einstecken einer Nadel angeordnet. Dabei ist es möglich, im Falle von zwei Fäden, von denen jeder mit einer Nadel fest verbunden ist, die Nadeln mit großem gegenseitigen Abstand in den Schlitzen der Faltkarte festzulegen, so daß jeder Faden separat ergriffen werden kann, ohne daß dabei der andere Faden mitgefaßt werden würde. Wenn das Nahtmaterial aus einem einzigen Faden besteht, der an jedem seiner Enden eine Nadel aufweist, kann in jedem der Schlitze einer der Nadeln festgelegt werden.

Im folgenden wird unter Bezugnahme auf die Zeichnungen ein Ausführungsbeispiel der Erfindung näher erläutert.

Es zeigen :

Figur 1 eine perspektivische Darstellung der Packung im geschlossenen Zustand,

Figur 2 einen Querschnitt durch die Packung,

Figur 3 das Aufreißen der Packung durch Auseinanderziehen der Folienteile an den Aufreißlaschen,

Figur 4 die Packung im geöffnetem Zustand nach dem Aufreißen,

Figur 5 eine perspektivische Darstellung des Zuschnitts der Faltkarte.

Figur 6 eine perspektivische Darstellung der Faltkarte während des Zusammenfaltens,

Figur 7 eine Ansicht der Faltkarte im zusammengefalteten Zustand.

Figur 8 eine Explosionsdarstellung der Teile der Packung,

Figur 9 eine Darstellung der Faltkarte mit mehreren darin enthaltenen Fäden,

Figur 10 eine ähnliche Darstellung wie Fig. 9, jedoch mit einem einzigen Faden, an dessen Ende sich eine gekrümmte Nadel befindet, und

Figur 11 eine ähnliche Darstellung wie Fign. 9 und 10, jedoch mit zwei Fäden, von denen jeder an seinem Ende eine gekrümmte Nadel aufweist bzw. mit einem Faden, der an jedem seiner Enden eine gekrümmte Nadel aufweist.

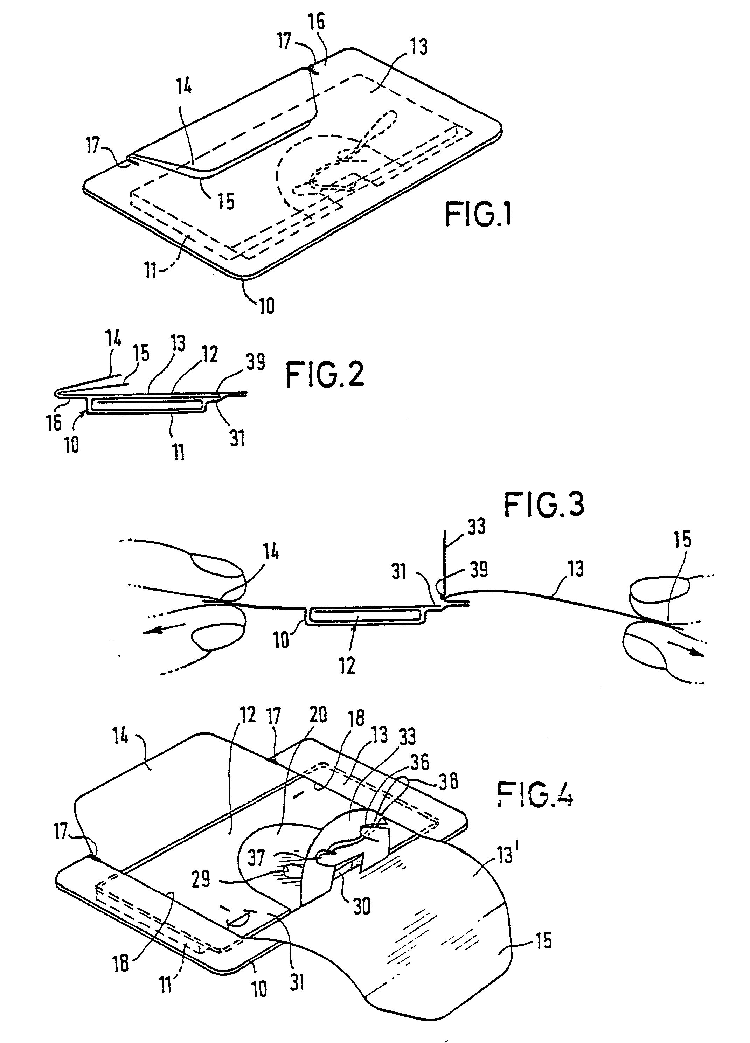

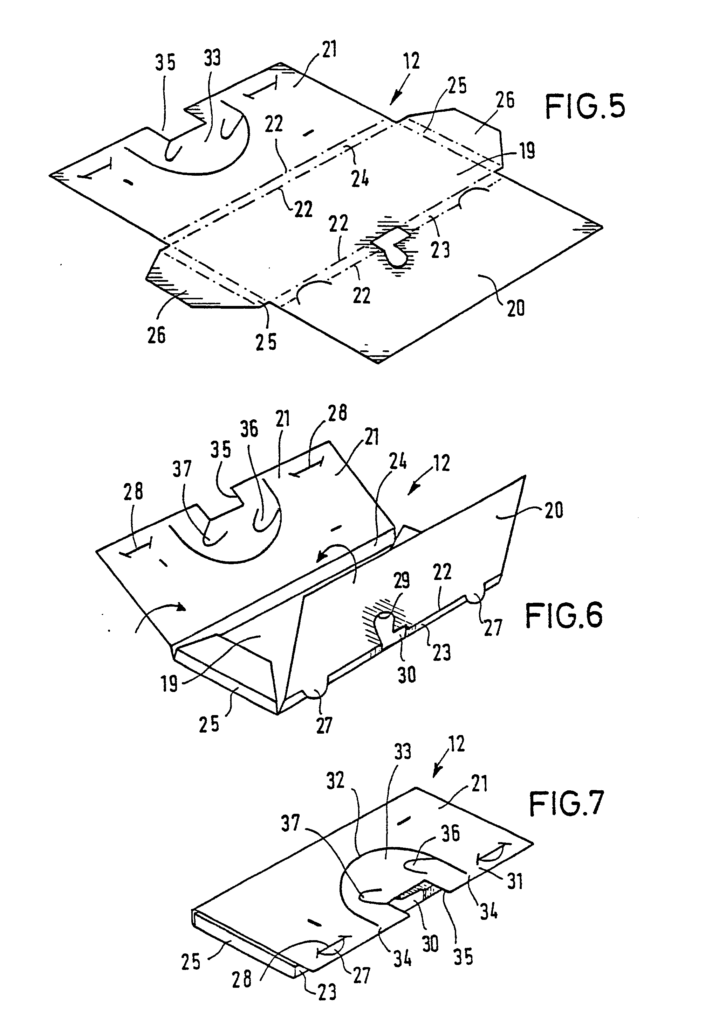

Die dargestelle Packung besteht aus einem ersten Folienteil 10 aus einer im wesentlichen steifen Folie, die so verformt ist, daß in ihr eine wannenförmige Vertiefung 11 zur Aufnahme der Faltkarte 12 gebildet wird, und dem zweiten Folienteil 13, das die Wanne 11 bedeckt und mit den um die Wanne 11 umlaufenden Rändern des ersten Folienteils 10 verschweißt ist. Die Packung hat im wesentlichen rechteckige Form, wobei das zweite Folienteil 13 eine ebenflächige Bedeckung für die in dem ersten Folienteil 10 ausgebildete Wanne 11 bildet. Beide Folienteile 11, 13 haben, angrenzend an eine Längskante der Packung, seitlich abstehende Aufreißlaschen 14, 15, die aufeinander passen und gemäß Fig. 1 über das zweite Folienteil 13 umgefaltet sind. Um die Aufreißlaschen 14, 15 besser vereinzeln zu können, ist die Aufreißlasche 15 ca. 2 mm breiter als die Aufreißlasche 14. An den Fußpunkten, an denen die Seitenkanten der Aufreißlaschen 14, 15 in den Rand 16 übergehen, befinden sich kurze Einschnitte 17 in den Materialien beider Folienteile. Diese Einschnitte 17 bewirken, daß das zweite Folienteil 13 beim Auseinanderziehen der Aufreißlaschen 14 und 15 gemäß Fig. 4 entlang zweier Reißlinien 18 aufreißt, welche nahezu parallel zu den Schmalseiten der Packung verlaufen, so daß über der Wanne 11 Folienstreifen aus dem zweiten Folienteil 13 stehen bleiben, welche die den Schmalseiten der Faltkarte 12 benachbarten Bereiche der Faltkarte auch bei geöffneter Packung in der Wanne 11 niederhalten, so daß die Faltkarte zwischen den Folienteilen 10, 13 in der Wanne 11 zurückgehalten wird.

In Fig. 5 ist der Zuschnitt der Faltkarte 12 dargestellt. Die Faltkarte 12 besteht aus Papier oder aus kunststoffbeschichtetem Papier oder ganz aus Kunststoff und weist eine Bodenwand 19, eine Zwischenwand 20 und eine Oberseite 21 auf. Die Bodenwand 19 hat einen rechteckigen Grundriß und ist entlang ihrer Längsseiten mit der Zwischenwand 20 und der Oberseite 21 jeweils über zwei parallele Faltlinien 22, die eine flächenförmige Schmalseite 23 bzw. 24 begrenzen, mit der Zwischenwand 20 und mit der Oberseite 21 verbunden. Von jeder Querseite der Bodenwand 19 steht über eine weitere Schmalseite 25 eine Faltlasche 26 ab.

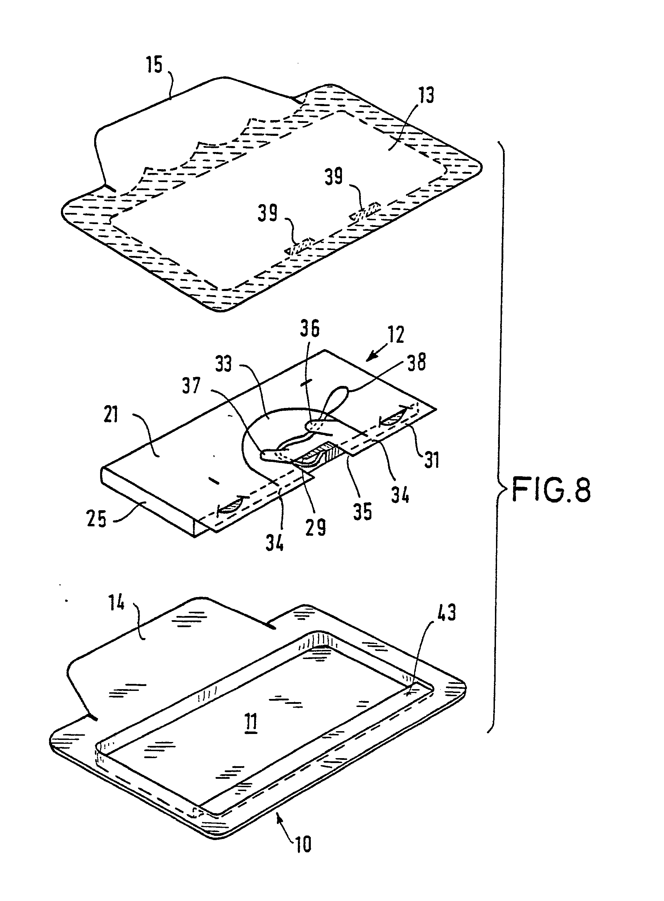

Beim Zusammenfalten der Faltkarte 12 werden die Schmalseiten 23, 24 und 25 von der Bodenwand 19 aus hochgefaltet, so daß sie die Seitenwände der fertigen kastenförmigen Faltkarte, die in Fig. 7 dargestellt ist, bilden. Dann wird die Zwischenwand 20 umgefaltet, die Faltlaschen 26 werden über die Zwischenwand 20 gefaltet und über die Faltlaschen 26 und die Zwischenwand 20 wird von der entgegengesetzten Seite her die Oberseite 21 gefaltet. Von der Zwischenwand 20 stehen ausgestanzte Zungen 27 ab, die über die angrenzende Faltlinie 22 hinausragen und somit nicht umgefaltet werden. Diese Zungen 27 werden durch entsprechende Schlitze 28 der Oberseite 21 hindurchgesteckt, um die Faltkarte 12 im geschlossenen Zustand zu halten. Alternativ wäre es auch möglich, die Oberseite 21 auf der Zwischenwand 20 festzukleben oder im Falle eines Kunstoffmaterials zu verschweißen.

In dem in Fig. 6 dargestellten Zustand der Faltkarte, also vor dem Schließen, wird das Nahtmaterial in den von der Faltkarte umschlossenen Raum eingelegt. Das Ende des Nahtmaterials wird durch eine Öffnung 29 der Zwischenwand 20 hindurchgeführt. Die Öffnung 29 weist eine sich in die Schmalseite 23 hinein erstreckende Verlängerung 30 auf.

Wie Fig. 7 zeigt, ist die Oberseite 21 breiter als die Bodenwand 19 bzw. die Zwischenwand 20, so daß das freie Ende der Oberseite 21 einen sich über die gesamte Länge der Faltkarte 12 erstreckenden frei überstehenden Rand 31 bildet. Im Mittelbereich der Länge der Oberseite 21 ist ein etwa halbkreisförmiger Schlitz 32 vorgesehen. Die Enden dieses Schlitzes 32 sind durch schmale Stege 34 von der freien Längskante des Randes 31 getrennt. Die Lasche 33 erstreckt sich also von dem Rand 31 aus in das Innere der Oberseite 21 hinein und liegt in einer Ebene mit dem übrigen Material der Oberseite 21. An dem Rand 31 und der Lasche 33 befindet sich eine rechteckige Aussparung 35 oberhalb der Verlängerung 30 der Öffnung 29.

Die Lasche 33 weist zwei ausgestanzte Zungen 36, 37 auf. Die Kante der Zunge 36 läuft in den Schlitz 32 hinein frei aus und die Kante der Zunge 37 läuft in die Aussparung 35 hinein frei aus. Die Zungen 36 und 37 dienen zum Festhalten des Endes des Nahtmaterials. Dies ist in Fig. 8 dargestellt. Das Nahtmaterial in Form eines Fadens 38 ragt durch die Öffnung 29 der Zwischenwand 20 hindurch und wird in dem Schlitz zwischen der Zunge 37 und der Lasche 33 eingeklemmt. Das Einführen des Nahtmaterials in den Schlitz erfolgt von der Aussparung 35 aus. Im weiteren Verlauf wird der Faden 38 in den zwischen der Zunge 36 und der Lasche 33 gebildeten Schlitz eingeführt und dort festgehalten. Die Faltkarte 12 wird in die Wanne 11 des ersten Folienteils 10 derart eingesetzt, daß der überstehende Rand 31 der Faltkarte 12 der Aufreißlasche 14 abgewandt ist. Die Wanne 11 ist so bemessen, daß die Faltkarte 12 genau hineinpaßt, wobei für die Aufnahme des Randes 31 ein Stufenabsatz 43 an dem ersten Folienteil 10 vorgesehen ist. Danach wird das zweite Folienteil 13 über das erste Folienteil 10 gelegt und die beiden Folienteile 10 und 13 werden längs ihrer Ränder verklebt oder verschweißt. Die Verbindungszonen sind in Fig. 8 an dem zweiten Folienteil 13 gestrichelt markiert. Die durch die Aussparung 35 getrennten Beine der Lasche 33 werden durch zwei Verbindungszonen 39, die sich von dem Rand des zweiten Folienteils 13 aus nach innen erstrecken, mit dem zweiten Folienteil verbunden. Diese Verbindungszonen 39 überragen die Stege 34 in Richtung auf die Längsmittellinie der Faltkarte.

Wenn zum Aufreißen der Packung gemäß Fign. 3 und 4 die nicht miteinander verbundenen frei abstehenden Aufreißlaschen 14 und 15 auseinandergezogen werden, reißt das zweite Folienteil 13 längs der Reißlinien 18, wodurch die Oberseite der Faltkarte 12 mit Ausnahme der endseitigen Randbereiche freigelegt wird. Wenn die Aufreißlinien 18 den gegenüberliegenden Rand erreicht haben, d. h. wenn der Rand 31 der Faltkarte freigelegt ist, zerreißen die Stege 34, da die Beine der Lasche 33 mit ihren Fußpunkten an dem abgerissenen Teil 13' des zweiten Folienteils 13 haften. Hierdurch stellt sich die Lasche 33 gemäß Fig. 4 aufrecht und bietet das an ihren Zungen 36 und 37 festgeklemmte Ende des Nahtmaterials 38 zum Entnehmen an. Durch das Hochstellen der Lasche 33 wird eine der Lasche entsprechende Aussparung der Oberseite 21 frei, wodurch eine entsprechende Fläche der Zwischenwand 20 freiliegt. In dieser freiliegenden Fläche befindet sich die Öffnung 29. Die Durchtrittsstelle des Nahtmaterials 38 verlagert sich von der Öffnung 29 in die Verlängerung 30 hinein.

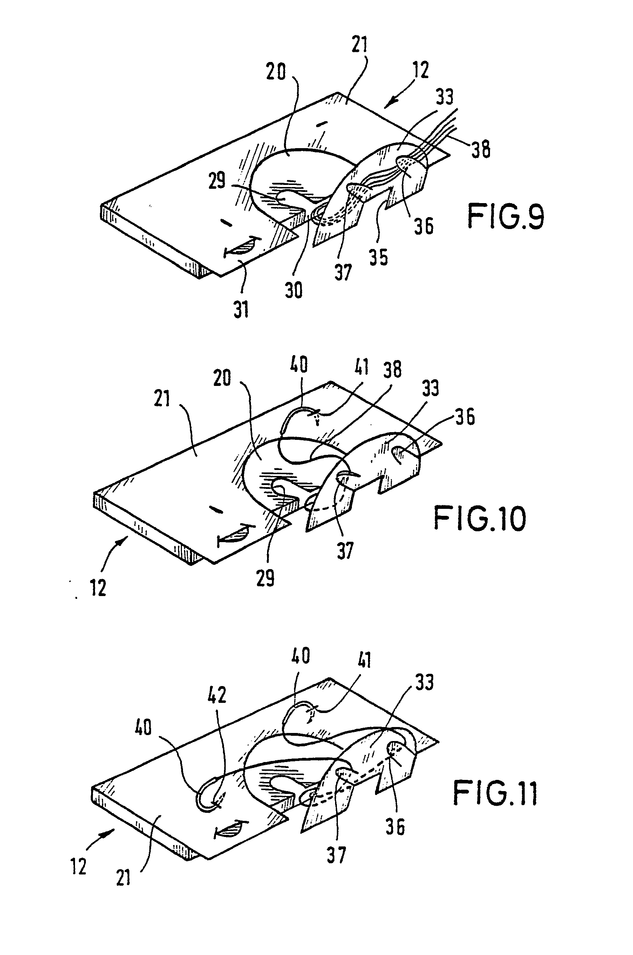

Die Fign. 9 bis 11 zeigen verschiedene Varianten der Anordnung von Nahtmaterial in bzw. an der Faltkarte 12, wobei die Lasche 33 jeweils in dem Zustand dargestellt ist, den sie nach dem Öffnen der Packung einnimmt. Hierbei sind aus Gründen der Übersichtlichkeit die beiden Folienteile 10 und 13 fortgelassen.

Bei dem Ausführungsbeispiel der Fig. 9 besteht das Nahtmaterial 38 aus zahlreichen parallelen Fäden, deren Endbereiche zwischen den Zungen 36 und 37 der Lasche 33 festgelegt sind. Diese Fäden führen bei noch geschlossener Faltkarte 12 durch die Öffnung 29 hindurch und sie sind von der Seite der Aussparung 35 her in den die Zunge 37 begrenzenden Schlitz eingeschoben, so daß sie durch diesen Schlitz hindurch auf die Oberseite der Lasche 33 gelangen. Von dort sind sie in den die Lasche 36 begrenzenden Schlitz eingezogen. Zwischen den beiden Zungen 37 und 36 verläuft das Nahtmaterial auf der Oberseite der Lasche 33, so daß es durch das durchsichtige zweite Folienteil 13 hindurch sichtbar ist. Nach dem Aufreißen der Packung können die einzelnen Fäden des Nahtmaterials 38 einzeln ergriffen und aus der Faltkarte 12 herausgezogen werden. Es ist auch möglich, die Lasche 33, die in dem in Fig. 9 dargestellten Zustand nur noch an dem (nicht dargestellten) zweiten Folienteil 13 haftet, von diesem zweiten Folienteil ganz abzureißen, um das gesamte Nahtmaterial herauszuziehen und die Lasche 33 als Halterung für das Nahtmaterial zu verwenden und die Packung fortzuwerfen. Auf der Lasche 33 können zur Kennzeichnung des an ihr festgeklemmten Nahtmaterials aufgedruckte Daten, Abbildungen, Farbkodierungen o. dgl. angebracht sein.

Fig. 10 zeigt eine Variante, bei der das Nahtmaterial aus einem einzigen Faden besteht, an dessen Ende eine Nadel 40 fest angebracht ist. Das Nahtmaterial 38 ist in der gleichen Weise aus dem Innern der Faltkarte herausgeführt wie in Fig. 9, jedoch ist der Faden nach seinem Durchtritt durch den Schlitz der Zunge 37 nicht noch unter die Zunge 36 geführt, sondern mit seiner Nadel 40 in einen Schlitz 41 eingesteckt, der in der Oberseite 21 der Faltkarte angeordnet ist. Beim Aufreißen der Packung stellt sich auch hier die Lasche 33 hoch. Das Nahtmaterial 38, das über den Rand der Lasche 33 hinweggeht, wird dabei ebenfalls angehoben, so daß das Nahtmaterial auf einfache Weise ergriffen werden kann.

Bei der in Fig. 11 dargestellten Variante wird zusätzlich zu dem Schlitz 41 noch ein weiterer Schlitz 42 auf der Oberseite 21 zum Festlegen einer zweiten Nadel 40 benutzt. Die Faltkarte 12 dient hierbei zur Aufnahme von zwei getrennten Fäden, von denen jeder eine Nadel 40 aufweist. Der eine Faden ist durch den Schlitz der Zunge 36 und der andere Faden ist durch den Schlitz der Zunge 37 hindurchgeführt. Beide Fäden verlaufen quer über den Rand der Lasche 33, so daß sie beim Hochstellen dieser Lasche 33 einzeln ergriffen werden können. Um das Ergreifen zu erleichtern, befinden sich die Schlitze 41 und 42 in unterschiedlichen Hälften der Oberseite 21, so daß sie möglichst große Abstände haben und die beiden Fäden sich in ihrem Verlauf außerhalb der Faltkarte nicht kreuzen. Anstelle der beiden Fäden kann auch ein einziger Faden mit zwei an seinen Enden befestigten Nadeln auf die beschriebene Weise festgelegt werden.

**Patentansprüche**

1. Packung für chirurgisches Nahtmaterial, mit einem abdichtend verschlossenen Behälter (10) aus einem wannenförmigen ersten Folienteil und einem das erste Folienteil entlang gegeneinanderliegender Randbereiche als aufreißbarer Deckel verschließenden zweiten Folienteil (13), mit an den Folienteilen vorgesehenen Aufreißlaschen (14, 15) und mit einer in dem Behälter angeordneten, die wesentliche Menge des Nahtmaterials enthaltenen Faltkarte (12), die eine an dem den Aufreißlaschen abgewandten Rand mit dem zweiten Folienteil (13) fest verbundene Lasche (33) mit einem Schlitz zum Festlegen eines Abschnitts des aus der Faltkarte herausragenden Nahtmaterials aufweist, dadurch gekennzeichnet, daß die ungefaltete Lasche (33) aus einem ausgestanzten Teil der nach dem Aufreißen des zweiten Folienteils (13) freiliegenden Oberseite (21) der Faltkarte (12) besteht und mit ihrem den Aufreißlaschen (14, 15) abgewandten Rand an dem zweiten Folienteil (13) derart befestigt ist, daß sie sich beim Aufreißen des zweiten Folienteils (13) aus der Oberseite der Faltkarte heraus hochstellt.

2. Packung nach Anspruch 1, dadurch gekennzeichnet, daß die Lasche (33) über schmale abreißbare Stege (34) mit der Oberseite (21) der Faltkarte (12) verbunden ist.

3. Packung nach Anspruch 2, dadurch gekennzeichnet, daß die Stege (34) an dem den Aufreißlaschen (14, 15) abgewandten Rand der Faltkarte (12) angeordnet sind.

4. Packung nach einem der Ansprüche 1 bis 3, dadurch gekennzeichnet, daß die Oberseite (21) der Faltkarte (12) mit dem den Aufreißlaschen (14, 15) abgewandten Rand (31) frei übersteht und daß der überstehende Bereich die Vertiefung der aus dem ersten Folienteil (10) gebildeten Wanne (11) überragt und im wesentlichen nur im Bereich der Lasche (33) mit dem zweiten Folienteil (13) verbunden ist.

5. Packung nach einem der Ansprüche 1 bis 4, dadurch gekennzeichnet, daß die Lasche (33) wenigstens annähernd halbkreisförmig ausgebildet und in der Mitte der Länge der Faltkarte (12) angeordnet ist.

6. Packung nach einem der Ansprüche 1 bis 5, dadurch egekennzeichnet, daß die Faltkarte (12) unterhalb ihrer Oberseite (21) eine den Raum zur Aufnahme des Nahtmaterials (38) nach oben begrenzende Zwischenwand (20) aufweist, die unter der Lasche (33) eine Öffnung (29) für den Durchtritt des an der Lasche (33) festgelegten Nahtmaterials (38) aufweist.

7. Packung nach Anspruch 6, dadurch gekennzeichnet, daß die Öffnung (29) sich bis in die benachbarte Schmalseite (23) der Faltkarte (12) hineinerstreckt.

8. Packung nach einem der Ansprüche 1 bis 7, dadurch gekennzeichnet, daß die Lasche (33) mindestens eine ausgestanzte Zunge (36, 37) aufweist, deren Kante in einen Rand der Lasche (33) ausläuft.

9. Packung nach einem der Ansprüche 1 bis 8, dadurch gekennzeichnet, daß die Lasche (33) mit zwei durch eine Aussparung (35) voneinander getrennten Beinen an dem zweiten Folienteil (13) befestigt ist.

10. Packung nach den Ansprüchen 8 und 9, dadurch gekennzeichnet, daß mindestens zwei Zungen (36, 37) vorgesehen sind, von denen eine in die Aussparung (35) auslaufende Kante aufweist.

11. Packung nach einem der Ansprüche 1 bis 10, dadurch gekennzeichnet, daß in Längsrichtung der Faltkarte (12) vor und hinter der Lasche jeweils ein Schlitz (41, 42) zum Einstecken der Nadel (40) angeordnet ist.

12. Packung nach einem der Ansprüche 1 bis 11, dadurch gekennzeichnet, daß die Faltkarte (12) kastenförmig ausgebildet ist und flächenförmige Schmalseiten (23, 24, 25) aufweist.

## Claims

1. Packaging for surgical suture material comprising a sealingly closed container (10) of a tub-shaped first sheet portion and a second sheet portion (13) closing the first sheet portion along marginal regions as a cover adapted to be torn open, rip flaps (14, 15) provided at the sheet portions, and a folding card (12) disposed in the container and including the substantial amount of suture material, which folding card comprises at the border averted from the rip flaps a flap (33) integrated with the first sheet portion (13) and having a slot for fixing a section of suture material extending out of the folding card, characterized in that the unfolded flap (33) consists of a stamped out part of the top side (21) of the folding card (12) exposed upon tearing open the second sheet portion (13), and that, with its border averted from the rip flaps (14, 15), said strap is fixed to the second sheet portion (13) so that it becomes erected from the top side of the folding card upon tearing open the second sheet portion (13).

2. Packaging as defined in claim 1, characterized in that, via narrow tearable stems (34), the flap (33) is connected to the top side (21) of the folding card (12).

3. Packaging as defined in claim 2, characterized in that the stems (34) are provided at the border of the folding card (12) averted from the rip flaps (14, 15).

4. Packaging as defined in one of claims 1 to 3, characterized in that the border (31) of the top side (21) of the folding card (12) averted from the rip flaps (14, 15) is freely salient and that the salient region projects above the recess of the tub (11) formed by the first sheet portion (10) and is connected to the second sheet portion (13) but substantially only within the range of the flap (33).

5. Packaging as defined in one of claims 1 to 4, characterized in that the flap (33) is shaped at least approximately semicircular and that it is provided in the center of the length of the folding card (12).

6. Packaging as defined in one of claims 1 to 5, characterized in that beneath its top side (21), the folding card (12) comprises a partition wall (20) limiting upwardly the space for accomodating the suture material (38) and containing beneath the flap (33) an aperture (29) for the passage of the suture material (38) fixed at the flap (33).

7. Packaging as defined in claim 6, characterized in that the aperture (29) extends as far as into the adjacent narrow side (23) of the folding card (12).

8. Packaging as defined in one of claims 1 to 7, characterized in that the flap (33) comprises at least one stamped out tongue (36, 37), whose edge ends in one border of the flap (33).

9. Packaging as defined in one of claims 1 to 8, characterized in that the flap (33) is fixed to the second sheet portion (13) by two legs spaced apart by a recess (35).

10. Packaging as defined in claims 8 and 9, characterized in that at least two tongues (36, 37) are provided of which one contains an edge ending in the recess (35).

11. Packaging as defined in one of claims 1 to 10, characterized in that in advance and behind the flap, in longitudinal direction of the folding card (12), there is provided a respective slot (41, 42) for the insertion of needle (40).

12. Packaging as defined in one of claims 1 to 11, characterized in that the folding card (12) is box-shaped with flat narrow sides (23, 24, 25).

## Revendications

1. Empaquetage pour matériaux de suture chirurgicale, comportant une boîte (10) fermée de manière étanche constituée d'une première feuille en forme d'un bac et d'une deuxième feuille (13) servant de couvercle arrachable, qui ferme la première feuille au niveau de leurs bords opposés, des pattes de déchirure (14, 15) ménagées sur les feuilles et une carte pliante (12) logée dans la boîte et renfermant la partie essentielle du matériau de suture, cette carte comportant au niveau du bord opposé aux pattes d'arrachement une patte (23) avec une fente solidaire de la deuxième feuille (13) pour fixer une partie du matériau de suture en saillie sur la carte pliante, caractérisé en ce que la patte (33) non pliée est constituée d'une partie découpée dans la partie supérieure (21) de la carte pliante (12) dégagée après l'ouverture par arrachement de la deuxième feuille (13) et qu'elle est fixée avec son bord opposé aux pattes d'arrachement (14, 15) à la deuxième feuille (13), en ce qu'elle se relève sur la partie supérieure de la carte pliante lors de la déchirure de la deuxième feuille (13).

2. Empaquetage selon la revendication 1, caractérisé en ce que la patte (33) est reliée à la partie supérieure (21) de la carte pliante (12) par des nervures minces à déchirer (34).

3. Empaquetage selon la revendication 2, caractérisé en ce que les nervures (34) sont disposées sur le bord de la carte pliante (12) opposé aux pattes d'arrachement (14, 15).

4. Empaquetage selon l'une quelconque des revendications 1 à 3, caractérisé en ce que la partie supérieure (21) de la carte pliante (12) est librement en saillie sur le bord (31) opposé aux pattes d'arrachement (14, 15) et en ce que la partie en saillie recouvre la cavité du bac (11) formé à partir de la première feuille (10), et qu'elle n'est essentiellement reliée qu'au niveau de la patte (33) à la deuxième feuille (13).

5. Empaquetage selon l'une quelconque des revendications 1 à 4, caractérisé en ce que la patte (33) est au moins approximativement en forme de demi-cercle et disposée au centre de la longueur de la carte pliante (12).

6. Empaquetage selon l'une quelconque des revendications 1 à 5, caractérisé en ce que la carte pliante (12) comporte au-dessous de sa partie supérieure (21) une cloison délimitant vers le haut l'espace destiné à recevoir le matériau de suture (38), qui comprend au-dessous de la patte (33) une ouverture (29) pour le passage du matériau de suture (38) fixé sur la patte (33).

7. Empaquetage selon la revendication 6, caractérisé en ce que l'ouverture (29) s'étend jusqu'à l'intérieur du petit côté contigu (23) de la carte pliante (12).

8. Empaquetage selon l'une quelconque des revendications 1 à 7, caractérisé en ce que la patte (33) comporte au moins une languette découpée (36, 37) dont l'arête se termine dans un bord de la patte (33).

9. Empaquetage selon l'une quelconque des revendications 1 à 8, caractérisé en ce que la patte (33) est fixée à la deuxième feuille (13) par deux branches séparées l'une de l'autre par un évidement (35).

10. Empaquetage selon les revendications 8 et 9, caractérisé en ce que deux languettes (36, 37) au moins sont prévues dont l'une comporte une arête se terminant dans l'évidement (35).

11. Empaquetage selon l'une quelconque des revendications 1 à 10, caractérisé en ce qu'une fente (41, 42) pour la fixation de l'aiguille (40) est disposée dans le sens longitudinal de la carte pliante (12) devant et derrière la patte.

12. Empaquetage selon l'une quelconque des revendications 1 à 11, caractérisé en ce que la carte pliante (12) est en forme de caisson et comporte des petits côtés plans (23, 24, 25).

0 115 580

FIG.1

FIG.2

FIG.3

FIG.4

1

FIG.5

FIG.6

FIG.7

15

13

39

39

39

36

12

33

38

37

21

31

25

34

35

34

29

34

FIG.8

14

43

11

10

FIG.9

FIG.10

FIG.11